# EUROPEAN PATENT SPECIFICATION

(11) **EP 1 235 611 B1**
(45) Date of publication and mention of the grant of the patent: **07.09.2005**
(21) Application number: 00991015.9
(22) Date of filing: 15.11.2000
(51) Int. Cl.: A61M 25/02

(54) **SKIN ATTACHMENT MEMBER**
BEFESTIGUNGSELEMENT FÜR DIE HAUT
ELEMENT DE FIXATION LA PEAU

(30) Priority: 15.11.1999 US 440384
(43) Date of publication of application: 04.09.2002
(73) Proprietor: VELCRO INDUSTRIES B.V., Curacao (AN)
(72) Inventor: KINGSFORD, Howard, A., Amherst, NH 03031 (US)
(74) Representative: Müller-Boré & Partner Patentanwälte
(86) International application number: PCT/US2000/042151
(87) International publication number: WO 2001/036037

(56) References cited:
- EP-A- 0 497 620
- WO-A-98/11937
- US-A- 3 964 482
- US-A- 4 133 307
- US-A- 4 164 943

## Description

### Background of the Invention

The invention relates to an attachment member for securing objects to skin.

A patch with an array of microneedles which penetrate into the stratum corneum is known. The microneedles are made out of silicon using the same etching process used to manufacture computer chips.

### Summary of the Invention

The attachment member of the invention, formed of plastic resin, includes a base layer from which extend an array of many tiny, integral skin-penetrating plastic elements having one or more barbs which lodge in the skin and resist removal of the attachment member. The member is configured such that the elements securely fasten to the skin without penetrating deeply enough to cause pain and discomfort.

According to one aspect of the invention, a skin attachment member of plastic resin includes a sheet-form backing, and an array of skin penetrating elements extending integrally from the backing. The skin penetrating elements are configured to penetrate into the epidermal skin layer and are sized to limit painful contact with nerves below the epidermal skin layer. At least many of the skin penetrating elements each includes at least one retention barb extending from an outer surface of the skin penetrating element. The barbs are configured to cooperate to resist removal of the skin attachment member from skin.

Embodiments of this aspect of the invention may include one or more of the following features.

Each skin penetrating element has a cone-shaped body. The base of the cone-shaped body has a diameter of about 0,08 mm 0.003 inch. Each skin penetrating element has a length of about 0.012" and a pointed tip. The backing has a thickness in a range of about 0,08 to 0,2 mm (0.003 to 0.008 inch).

In an illustrated embodiment, the retention barb is located about 0,2 to 0,2413 mm (0.008 to 0.0095 inch) along a length of the skin penetrating element from the backing, has a length of about 0,00254 mm (0.0001 inch), and tapers from a thickness of about 0,00254 mm (0.0001 inch) to a point at an angle of about 72 °. Each skin penetrating element includes, e.g., two barbs.

The skin attachment member has a density of about 400 skin penetrating elements in a 6,4516 mm² (0.01 in²) area, i.e., 61,957/mm² (40,000/in²). The skin penetrating elements are spaced apart from each other a distance of about 0.003".

The skin attachment member is formed from nylon, polyethylene teraphthalate, or polyester. The skin attachment member is formed by molding.

In an illustrated embodiment, at least many of the skin penetrating elements define at least one groove in an outer surface of the skin penetrating elements. The skin penetrating elements are oriented perpendicular to the backing.

Other features and advantages of the invention will be apparent from the following description, and from the claims.

### Brief Description of the Drawings

Fig. 1 is a highly magnified side view of a section of a skin attachment member according to the invention shown secured in the epidermis;
Fig. 2 is a diagrammatic perspective view of the section of the skin attachment member of Fig. 1;
Fig. 3A is a side view of a skin-penetrating element of the skin attachment member of Fig. 1;
Fig. 3B is a another side view of the element of Fig. 3A, rotated 90 degrees relative to Fig. 3A;
Fig. 3C is an end view of the element of Fig. 3A taken along lines 3C-3C in Fig. 3A;
Fig. 3D is a cross-sectional view of the element of Fig. 3A taken along lines 3D-3D in Fig. 3B;
Fig. 4 is a perspective view of section A of Fig. 3B showing a barb of the element of Fig. 3A;
Fig. 5 shows an alternative embodiment of a barb;
Fig. 6A is a diagrammatic representation of a molding machine for forming the skin attachment member of Fig. 1;
Fig. 6B shows a mold roll, pressure roll, and trim roller of the molding machine of Fig. 6A;
Fig. 6C is an enlarged view of the mold roll and pressure roll of the molding machine;
Fig. 7A is a side view of the mold roll of Fig. 6A;
Fig. 7B is a cross-sectional view of the mold roll, taken along lines 7B-7B in Fig. 7A;
Fig. 7C is an end view of the mold roll, taken along lines 7C-7C in Fig. 7B;
Fig. 7D is a magnified side view of portion 7D of the mold roll of Fig. 7A;
Fig. 7E shows laser machining of the mold roll; and
Fig. 8 shows an alterative edge formation on a mold roll.

### Description of the Preferred Embodiments

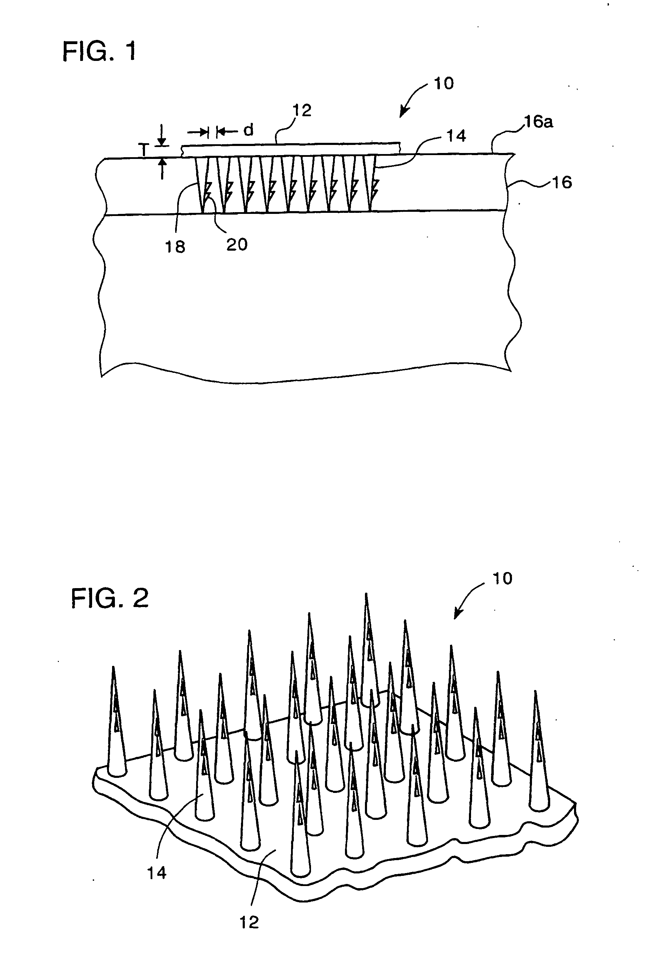

Referring to Figs. 1 and 2, a skin attachment member 10, formed of plastic resin, includes a backing 12 and multiple, parallel rows of integrally molded, pointed projections or elements 14 extending from backing 12 for penetrating into the epidermis 16. The skin-penetrating elements 14 each include a cone-shaped body 18 with one or more discrete barbs 20 extending from the body for securing skin attachment member 10 to epidermis 16.

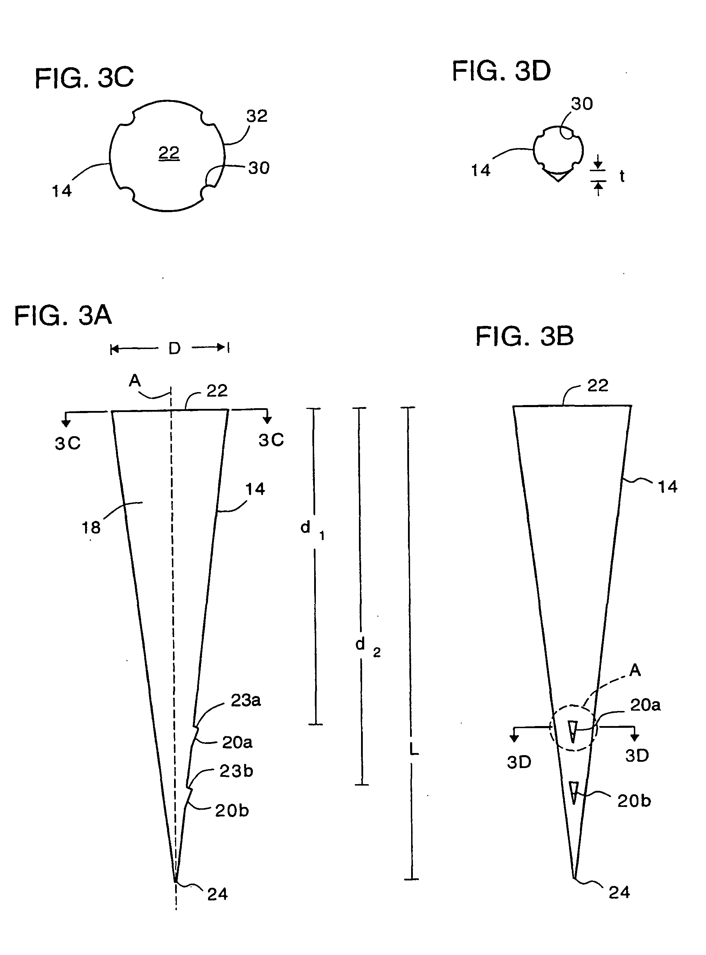

The length of elements 14 is selected such that they do not penetrate so far into the skin as to contact nerves located below the outer layers of the epidermis, as to cause significant pain and discomfort, but are long enough to cooperate with each other to provide sufficient adhesion to the skin. Elements 14 can be sized to extend into the portion of skin lying below the stratum corneum layer of the skin because of the small size of elements 14 and the spacing between nerves at this depth. For example, referring also to Fig. 3A, plastic elements 14 have a length, L. of about 3, 6576 mm (0.012 inch). Since the thickness of the epidermis varies, for example, with age, the location on skin, and the gender of the patient, the length of elements 14 can be selected for the particular use.

Cone 18 tapers from a larger diameter proximal base 22 to a distal pointed tip 24. The conical shape and sharp point of elements 14 ease their penetration into epidermis 16. The diameter of base 22 is selected to be large enough to help prevent breaking off of the projecting elements 14 from backing 12, while limiting the size of the opening made in the outer surface 16a of epidermis 16. For example, cone 18 has a base diameter, D, of about 0,9144 mm (0.003 inch). Backing 12 has a thickness, T, in a range of about 0.003" to 0.008" to provide member 10 with sufficient handling characteristics.

The skin-penetrating projecting plastic elements 14 can be other than conical in shape. For example, elements 14 can be in the shape of a pyramid, a tetrahedron, or may be elliptical or square in cross-section, tapering to points at their distal ends. Rather than taper distally, elements 14 can progressively step down in diameter. Regardless of the particular shape selected, the elements 14 include sharp pointed tips 24 to ease tissue penetration.

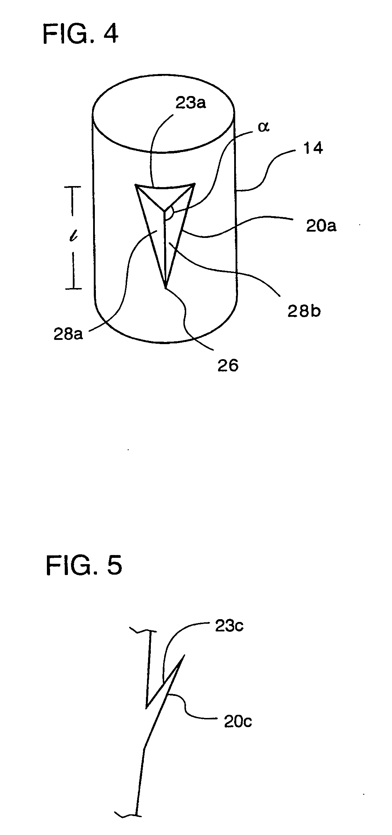

Referring to Figs. 4A and 4B, projecting elements 14 are shown with two discrete barbs 20a, 20b for retaining elements 14 in the skin, though fewer or more barbs can be disposed on cone 28 to provide the desired retention characteristics. The location of barbs 20 can be selected to take advantage of the greater elasticity of the skin portions lying below the stratum corneum to provide greater holding force. For example, barb 20a has a top surface 23a located a distance, d₁, of about 2,4384 mm (0.008 inch) from base 22, and barb 20b has a top surface 23b located a distance, d₂, of about 2,8956 mm (0.0095 inch) from base 22.

Referring to Fig. 4, which is an enlarged view of section A of Fig. 3B, barbs 20a. 20b are roughly half-pyramids, each having a flat upper surface 23a which is perpendicular to a longitudinal axis, A, of the projecting element 14, and sloped sides 28a, 28b. Barbs 20a, 20b have a length, I, of about 0,03048 mm (0.0001 inch); and a thickness, t (Fig. 3d), of about 0,03048 mm (0.0001 inch), which tapers to a point 26 at an angle, α, of about 72°.

In use, due to the elasticity of the skin, member 10 is secured to skin surface 16a by surrounding of the barbs by the epidermis. To improve retention of member 10 to the skin, the barbs can be angled as shown in Fig. 5. Here, a barb 20c has a sloped upper surface 23c.

The density of projecting elements 14 on backing 12 depends on use. For example, in high strain applications, a higher density provides better skin attachment, whereas, in applications in which member 10 is not subjected to high strain, a lower density is better for limiting the possibility of inflammation. If the density is too high, it can require too much force for elements 14 to penetrate into the skin. A density of 400 projecting elements in a 64,516 mm² (0.1 in²) area provides good skin attachment while not requiring excessive insertion force. In this case the projecting elements 14 are preferably spaced apart a distance, d (Fig. 1), of about 0,9144 mm (0.003 inch).

Member 10 and its projecting elements 14 are preferably formed from a thermoplastic, biocompatible polymer, which is stiff enough to penetrate skin but not brittle, and capable of filling a mold and retaining its molded form. Example of suitable polymers include nylon, polyethylene teraphthalate, and polyester.

Referring to Fig. 3C and 3D, if desired for use in, for example, drug delivery, the projecting elements 14 include longitudinal grooves 30 in an outer surface 32 of cone 18, here four grooves 30 being shown, which provide passages for drug delivery. The penetration of elements 14 into the epidermis facilitates the delivery of drugs through the epidermis by reducing the thickness of the skin barrier to the vascular layer below the epidermis.

Other uses of member 10 include securing an intravenous or other catheter to the skin, such as securing in place a port for peritoneal dialysis, thus replacing a suture or a butterfly or acting secondary to a suture, acting as bandaid type wound closure to hold two sections of skin together, as wound covering, as a delivery system for self-delivery of drugs, in vetinary applications, as a reaction indicator, for time release vaccination, in agricultural applications such as bundle ties limiting damage to produce, and in geotextile applications. Incorporated in the polymer from which member 10 is formed can be bacteria killing agents or medication.

Skin attachment member 10 can be molded as a continuous strip 10' according to the principles described in Fischer, U.S. Patent 4,794,028, hereby incorporated by reference.

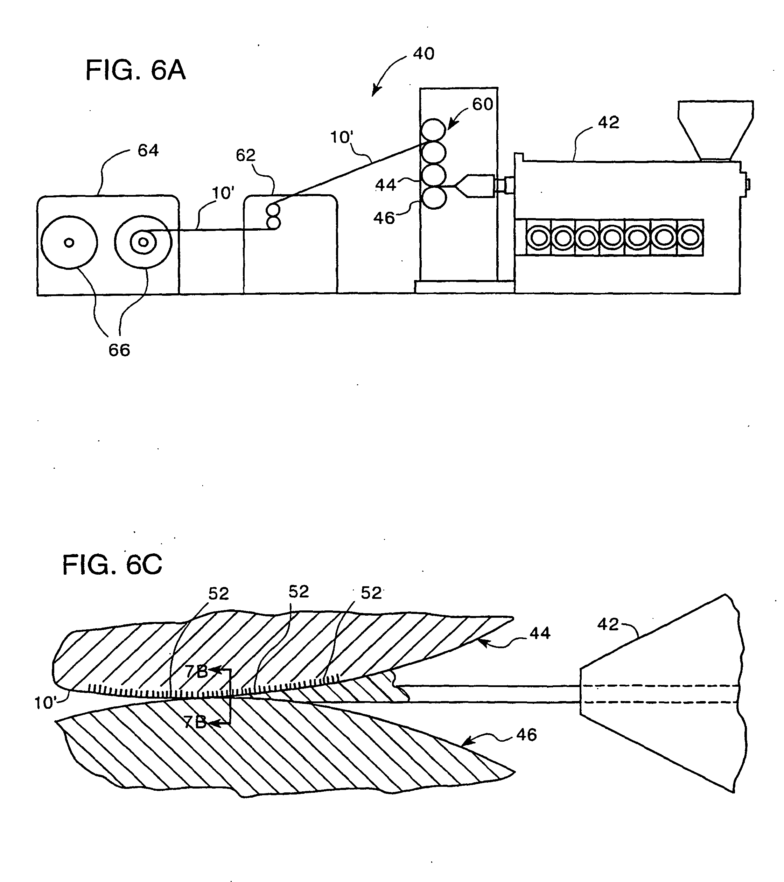

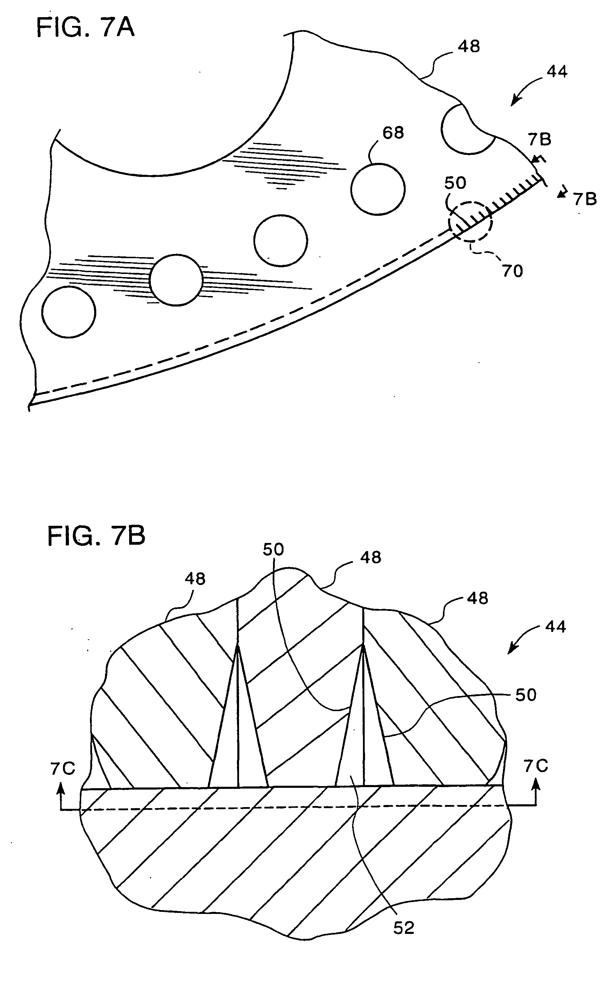

For example, referring to Figs. 6A-6C, a molding machine 40 includes an extruder 42 which delivers an extrusion of molten plastic material between a pair of rollers 44, 46 mounted for rotation in opposite directions. Roller 44 is a cooled mold roll having a set of stacked parallel plates 48 (Fig. 7B) in which edge formations 50 define rows of projecting element-mold cavities 52. Roller 46 is a pressure roll which coacts with mold roll 44 for formation of continuous strip 10'.

Molten resin is continuously extruded and applied with pressure against mold roll 44 using pressure roll 46. Molten resin is forced into mold cavities 52 and between rolls 44, 46, to form the projecting elements 14 integral with backing 12. After cooling while on the roll, the continuous strip 10' is stripped from mold roll 44, the projecting elements 14 undergoing temporary elastic deformation to achieve release from the mold cavities 52.

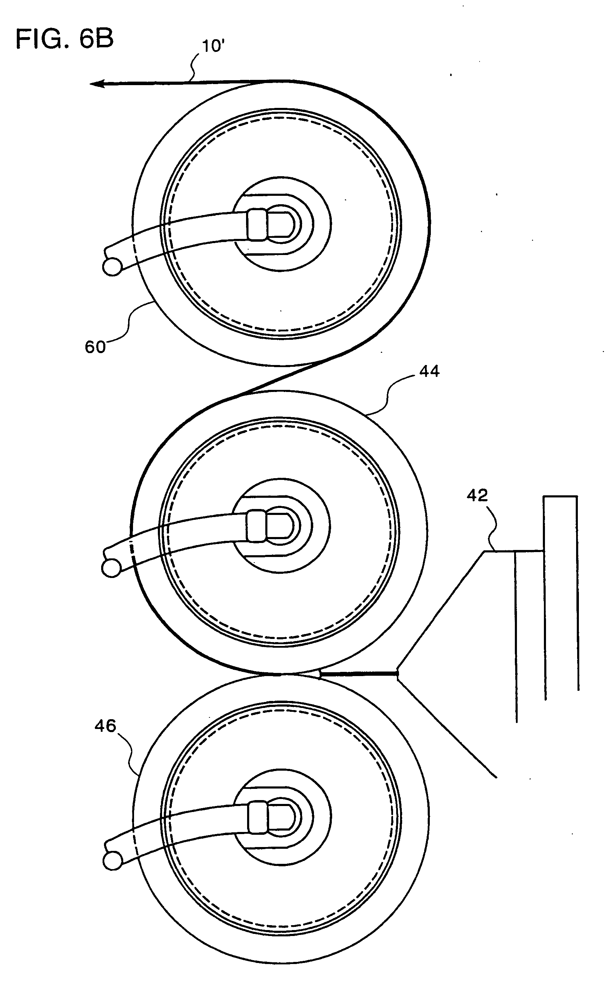

A trim roller assembly 60 is mounted above mold roll 44 such that continuous strip 10' is removed or stripped from mold roll 44 immediately upstream of trim roller assembly 60. Trim roller assembly 60 can include two rollers, as shown Fig. 6A, or one roller, as shown in Fig. 6B. A tensioning roller assembly 62 creates tension in member 10 for effecting the removal of strip 10' from mold roll 64. Downstream of tensioning roller assembly 62 is a winder 64 for winding continuous strip 10' on spools 66 for subsequent shipment, storage and use.

Referring to Figs. 7A and 7B, mold roll 44 includes a series of stacked plates 48 having edge formations 50 on either side of each plate. When stacked, plates 48 together define projecting element-forming cavities 52 within which projecting elements 14 are formed. Plates 48 also define water passages 68 for cooling of member 10'.

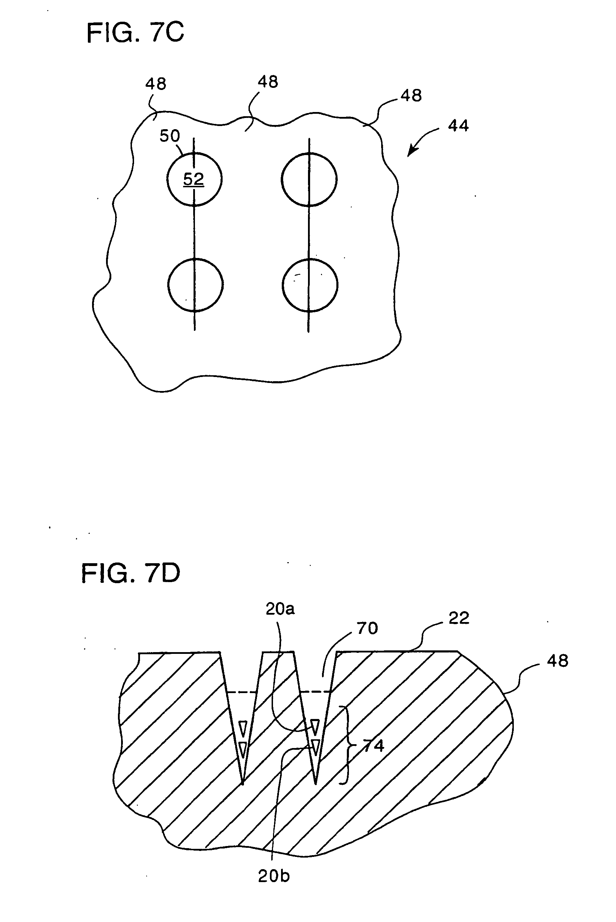

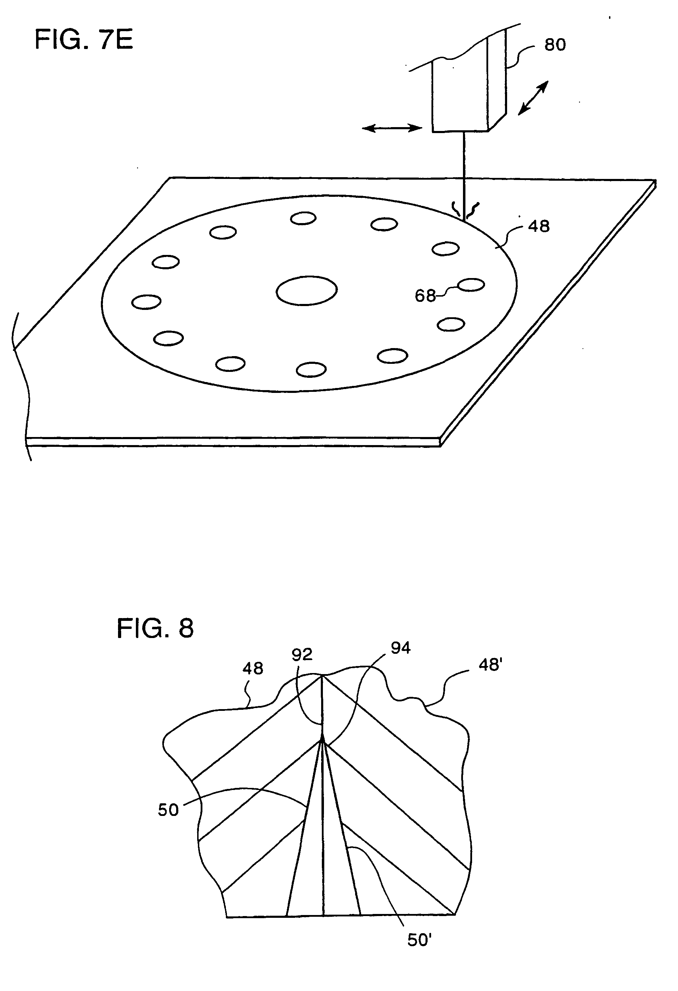

Referring to Fig. 7D, which is an enlarged view of section 7D of Fig. 7A, plates 48 of mold role 46 can be formed by etching a cone shape 70 for a length of about 0.004" from roll edge 72. The remaining tip portion 74 of the cone is formed by laser machining (Fig. 7E) in which a laser 80. under computer position control, is used to remove material from the plate to form the tip portion. Barb impressions 20a' and 20b' can also be formed using laser machining. The laser can also be controlled to form grooves 30 in cone 18. The mold rolls are preferably formed of beryllium copper, the temperature of which is controlled during molding such that the resin does not cool too fast during application.

Fig. 8 shows an alternative embodiment of a mold cavity in which a first plate 48 defines an edge formation 50 as described above, and a second plate 48' defines an edge formation 50' having a tip 94 terminating prior to a tip 92 of edge formation 50. Thus, distal tip 24 of element 12 is defined by tip 92.

While skin attachment member 10 has been described as including multiple, parallel rows of projecting elements 14, the mold rolls can be arranged such that the rows of elements 14 are offset or otherwise distributed on backing 12. The projecting elements 14 can also be formed such that their longitudinal axes are not perpendicular to backing 12 or are distributed at various angles to backing 12. While enough of the projecting elements 14 should include barbs 20 to provide the desired degree of securement to the skin, not all of projecting elements 14 need include barbs 20.

Other embodiments are within the scope of the following claims.

## Claims

1. A skin attachment member (10) of plastic resin, comprising:
a sheet-form backing (12), and
an array of skin penetrating elements (14) extending integrally from the backing (12), the skin penetrating elements being configured to penetrate into the epidermal skin layer (16) and sized to limit painful contact with nerves below the epideanal skin layer;
the skin penetrating elements (14) each including a body (18) extending from the backing, and a discrete retention barb (20a) extending from the body, the barb configured to cooperate to resist removal of the skin attachment member (10) from skin (16),
**characterized in that** the skin penetrating elements (14) are molded integrally with the backing (12) from a thermoplastic polymer.

2. The skin attachment member (10) of claim 1 wherein the body (18) of each skin penetrating element (14) is cone-shaped.

3. The skin attachment member (10) of claim 2 wherein the cone-shaped body (18) has a base (22) with a diamcter (D) of about (0.003 inch) 0.08 mm.

4. The skin attachment member (10) of claim 1 wherein each skin penetrating element (14) includes a pointed tip (24).

5. The skin attachment member (10) of claim 1 wherein each skin penetrating element (14) has a length (L) of about 0.012 inch (0.3 mm).

6. The skin attachment member (10) of claim 1 wherein the backing (12) has a thickness (T) in a range of about (0.003 to 0.008 inch), 0.08 to 0.2 mm.

7. The skin attachment member (10) of claim 1 wherein the retention barb (20a) of each skin penetrating element (14) is located a distance (d₁) of about (0.008 to 0.0095 inch) 0.2 to 0.24 mm along a length (L) of the skin penetrating element from the backing (12).

8. The skin attachment member (10) of claim 1 wherein the retention barb (20a) of each skin penetrating element (14) has a length (1) of about (0.0001 inch) 0.003 mm

9. The skin attachment member (10) of claim 1 wherein the retention barb (20a) of each skin penetrating element tapers from a thickness (t) of about (0.0001 inch) 0.003 mm to a point (26) at an angle (α) of about 72 degrees.

10. The skin attachment member (10) of claim 1 wherein each skin penetrating element (14) includes a second barb (20b).

11. The skin attachment member (10) of claim 1 having a density of about 400 skin penetrating elements (14) in a (0.1 inch²) 65 mm² area.

12. The skin attachment member (10) of claim 1 wherein the skin penetrating elements (14) are spaced apart from each other a distance (d) of about (0.003 inch) 0.08 mm.

13. The skin attachment member of claim 1 formed from nylon or polyethylene teraphthalate or polyester.

14. The skin attachment member (10) of claim 1 wherein at least many of the skin penetrating elements (14) define a groove (30) in an outer surface of the body (18).

15. The skin attachment member (10) of claim 1 wherein the skin penetrating elements (14) are oriented perpendicular to the backing (12).

## Patentansprüche

1. Befestigungselement (10) für die Haut aus Kunstharz, umfassend:
ein blattförmiges Rückseitenelement (12) und
ein Feld bzw. Bereich von hautpenetrierenden Elementen (14), die sich integral bzw. einstückig von dem Verstärkungs- bzw. Rückseitenelement (12) erstrecken, wobei die hautpenetrierenden Elemente konfiguriert sind, um in die epidermale Hautschicht (16) einzudringen, und dimensioniert sind, um einen schmerzlichen Kontakt mit Nerven unter der epidermalen Hautschicht
zu begrenzen,
wobei die hautpenetrierenden bzw. -durchdringenden Elemente (14) jeweils einen Körper (18), der sich von dem Rückseitenelement erstreckt, und einen gesonderten Rückhaltestachel bzw. -widerhaken (20a) beinhaltet, der sich von dem Körper erstreckt, wobei der Widerhaken konfiguriert ist, um einer Entfernung des Hautfestlegungsglieds bzw. -elements (10) von der Haut (16) zu widerstehen,
**dadurch gekennzeichnet, daß** die hautpenetrierenden Elemente (14) einstückig mit dem Rückseitenelement (12) aus einem thermoplastischen Polymer geformt sind.

2. Befestigungselement (10) für die Haut nach Anspruch 1, wobei der Körper (18) von jedem hautpenetrierenden Element (14) konusförmig ist.

3. Befestigungselement (10) für die Haut nach Anspruch 2, wobei der konusförmige Körper (18) eine Basis (22) mit einem Durchmesser (D) von etwa (0,003 Zoll) 0,08 mm aufweist.

4. Befestigungselement (10) für die Haut nach Anspruch 1, wobei jedes hautpenetrierende Element (14) eine zugespitzte Spitze (24) beinhaltet.

5. Befestigungselement (10) für die Haut nach Anspruch 1, wobei jedes hautpenetrierende Element (14) eine Länge (L) von etwa 0,012 Zoll (0,3 mm) aufweist.

6. Befestigungselement (10) für die Haut nach Anspruch 1, wobei das Rückseitenelement (12) eine Dicke (T) in einem Bereich von etwa (0,003 bis 0,008 Zoll) 0,08 bis 0,2 mm aufweist.

7. Befestigungselement (10) für die Haut nach Anspruch 1, wobei der Rückhaltewiderhaken (20a) von jedem hautpenetrierenden Element (14) in einem Abstand (d₁) von etwa (0,008 bis 0,0095 Zoll) 0,2 bis 0,24 mm entlang der Länge (L) des hautpenetrierenden Elements von dem Rückseitenelement (12) angeordnet ist.

8. Befestigungselement (10) für die Haut nach Anspruch 1, wobei der Rückhaltewiderhaken (20a) von jedem hautpenetrierenden Element (14) eine Länge (I) von etwa (0,0001 Zoll) 0,003 mm aufweist.

9. Befestigungselement (10) für die Haut nach Anspruch 1, wobei der Rückhaltewiderhaken (20a) von jedem hautpenetrierenden Element sich von einer Dicke (t) von etwa (0,0001 Zoll) 0,003 mm zu einer Spitze (26) unter einem Winkel (α) von etwa 72 Grad verjüngt.

10. Befestigungselement (10) für die Haut nach Anspruch 1, wobei jedes hautpenetrierende Element (14) einen zweiten Widerhaken (20b) umfaßt.

11. Befestigungselement (10) für die Haut nach Anspruch 1, das eine Dichte von etwa 400 hautpenetrierenden Elementen (14) in einer Fläche von (0,1 Zoll²) 65 mm² aufweist.

12. Befestigungselement (10) für die Haut nach Anspruch 1, wobei die hautpenetrierenden Elemente (14) voneinander um einen Abstand (d) von etwa (0,003 Zoll) 0,08 mm beabstandet sind.

13. Befestigungselement für die Haut nach Anspruch 1, gebildet aus Nylon oder Polyethylen-Teraphthalat oder Polyester.

14. Befestigungselement (10) für die Haut nach Anspruch 1, wobei wenigstens zahlreiche der hautpenetrierenden Elemente (14) eine Nut (30) in einer Außenoberfläche des Körpers (18) definieren.

15. Befestigungselement (10) für die Haut nach Anspruch 1, wobei die hautpenetrierenden Elemente (14) senkrecht zu der Rückseite (12) ausgerichtet sind.

## Revendications

1. Élément (10) de fixation à la peau en résine plastique comprenant :
un support (12) en forme de feuille, et
un ensemble d'éléments (14) pénétrant dans la peau s'étendant intégralement depuis le support, les éléments pénétrant dans la peau étant configurés pour pénétrer dans la couche épidermique (16) de la peau et dimensionnés pour limiter le contact douloureux avec les nerfs en dessous de la couche épidermique de la peau,
les éléments (14) pénétrant dans la peau comprenant chacun un corps (18) s'étendant depuis le support, et une barbe de rétention (20a) discrète s'étendant depuis le corps, la barbe étant configurée pour contribuer à résister au retrait de l'élément (10) de fixation à la peau hors de la peau (16),
**caractérisé en ce que** les éléments (14) pénétrant dans la peau sont moulés intégralement avec le support (12) à partir d'un polymère thermoplastique.

2. Élément (10) de fixation à la peau de la revendication 1 dans lequel le corps (18) de chaque élément (14) pénétrant dans la peau est en forme de cône.

3. Élément (10) de fixation à la peau de la revendication 1 dans lequel le corps (18) en forme de cône a une base (22) ayant un diamètre (D) d'environ 0,08 mm (0,003 pouce).

4. Élément (10) de fixation à la peau de la revendication 1 dans lequel chaque élément (14) pénétrant dans la peau comprend un bout pointu (24).

5. Élément (10) de fixation à la peau de la revendication 1 dans lequel chaque élément (14) pénétrant dans la peau a une longueur (L) d'environ 0,3 mm (0,012 pouce).

6. Élément (10) de fixation à la peau de la revendication 1 dans lequel le support (12) a une épaisseur (T) de l'ordre d'environ 0,08 à 0,2 mm (0,003 à 0,008 pouce).

7. Élément (10) de fixation à la peau de la revendication 1 dans lequel la barbe de rétention (20a) de chaque élément (14) pénétrant dans la peau est située à une distance (d₁) d'environ 0,2 à 0,24 mm (0,008 à 0,0095 pouce) le long d'une longueur (L) de l'élément pénétrant dans la peau par rapport au support (12).

8. Élément (10) de fixation à la peau de la revendication 1 dans lequel la barbe de rétention (20a) de chaque élément (14) pénétrant dans la peau a une longueur (I) d'environ 0,003 mm (0,0001 pouce).

9. Élément (10) de fixation à la peau de la revendication 1 dans lequel la barbe de rétention (20a) de chaque élément (14) pénétrant dans la peau s'effile depuis une épaisseur (t) d'environ 0, 003 mm (0,0001 pouce) en une pointe (26) à un angle (α) d'environ 72 degrés.

10. Élément (10) de fixation à la peau de la revendication 1 dans lequel chaque élément (14) pénétrant dans la peau comprend une deuxième barbe (20b).

11. Élément (10) de fixation à la peau de la revendication 1 ayant une densité d'environ 400 éléments (14) pénétrant dans la peau sur une surface de 65 mm² (0,1 pouce²).

12. Élément (10) de fixation à la peau de la revendication 1 dans lequel les éléments (14) pénétrant dans la peau sont espacés les uns des autres d'une distance (d) d'environ 0,08 mm (0,003 pouce).

13. Élément (10) de fixation à la peau de la revendication 1 formé de nylon ou de téréphtalate de polyéthylène ou de polyester.

14. Élément (10) de fixation à la peau de la revendication 1 dans lequel au moins un grand nombre des éléments (14) pénétrant dans la peau définissent une rainure (30) dans une surface extérieure du corps (18).

15. Élément (10) de fixation à la peau de la revendication 1 dans lequel les éléments (14) pénétrant dans la peau sont orientés perpendiculairement au support (12).
